# EUROPEAN PATENT SPECIFICATION

(11) **EP 1 062 318 B1**
(45) Date of publication and mention of the grant of the patent: **28.04.2004**
(21) Application number: 99906073.4
(22) Date of filing: 10.02.1999
(51) Int. Cl.: C12N 1/21, C12N 15/75

(54) **A PROKARYOTIC CELL COMPRISING TWO COPIES OF A GENE TRANSCRIBED IN DIFFERENT DIRECTIONS**
PROKARYONTISCHE ZELLE, DIE ZWEI KOPIEN EINES GENES ENTHÄLT, DIE IN ZWEI VERSCHIEDENE RICHTUNGEN TRANSKRIBIERT WERDEN.
CELLULE PROCARYOTE COMPRENANT DEUX COPIES D'UN GENE TRANSCRITES DANS DES DIRECTIONS DIFFERENTES

(30) Priority: 12.02.1998 DK 20198
(43) Date of publication of application: 27.12.2000
(73) Proprietor: Novozymes A/S, 2880 Bagsvaerd (DK)
(72) Inventor: JOERGENSEN, Steen Troels, Novo Nordisk a/s, DK-2880 Bagsvaerd (DK)
(86) International application number: PCT/DK1999/000064
(87) International publication number: WO 1999/041358

(56) References cited:
- EP-A- 0 284 126
- US-A- 5 460 954

## Description

### FIELD OF INVENTION:

A prokaryotic cell expressing a gene of interest and comprising at least two copies of said gene on the chromosome.

### BACKGROUND OF THE INVENTION:

Prokaryotic multicopy production cells, i.e. cells comprising more than one copy of a gene of interest, have been used for production of proteins of interest at industrial scale.

Preferred multicopy production cells are cells which stabile maintain the individual copies of the genes of interest during fermentation.

Further, due to environmental concerns there is an increasing desire for production cells which do not comprise any integrated antibiotic resistance genes on the chromosome and according to this line production cells which are capable of stabile maintaining the copies of the gene of interest in a fermentation medium NOT comprising an antibiotic.

EP 284126 describes a solution to the stability issue above by providing a method for constructing a prokaryotic cell comprising on the chromosome at least two copies of a gene of interest separated by endogenous DNA, which is vital (essential) to the host cell (see claim 1 of EP 284126).

The individual copies of the gene of interest are stabile maintained in a fermented cell population due to the essential DNA. If a cell crosses out this vital DNA by homologous recombination between the two copies of the gene of interest, the cell looses vital DNA and this specific cell will die.

Thereby it is possible to maintain a stable cell population comprising the copies of the gene of interest.

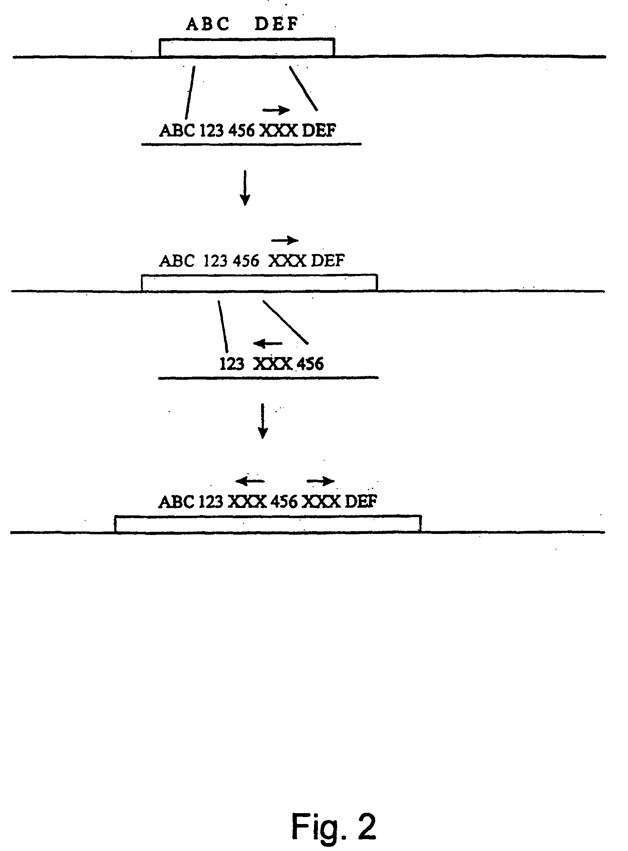

The method requires a knowledge of which DNA regions are vital to the cell. Alternatively, the gene is integrated on very distant places in the chromosome to have a high probability that there would be vital DNA between the copies (see figure 2 of EP 284126). This is a relatively laborious process and uncertain process.

### SUMMARY OF THE INVENTION:

The problem to be solved by the present invention is to provide a prokaryotic cell expressing a gene of interest and comprising at least two copies of said gene on the chromosome.

Further, said prokaryotic cell should be
a) stable in the sense that during fermentation it is able to maintain two copies of the gene on the chromosome; and
b) able to stably maintain the two copies of the gene without having a DNA segment, situated between the two copies of the gene, which segment is essential for the growth of the cell as described in e.g. EP 284126.

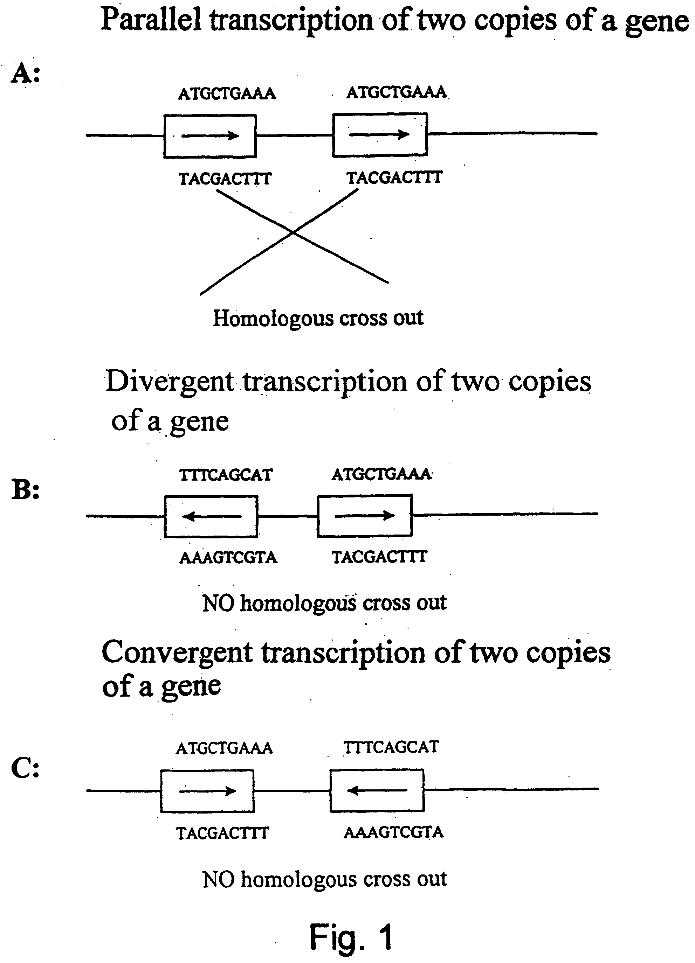

The solution is based on that the present inventor has identified that it is possible during fermentation to stably maintaining **two anti-parallelly** transcribed copies of a gene on the chromosome of a cell (see figure 1) and the two anti-parallelly transcribed genes can be **stably maintained** even **without having a DNA segment,** situated between the two copies of the gene, which is **essential** for the growth of the cell.

Accordingly, in a first aspect the present invention relates to a prokaryotic cell expressing a gene of interest characterised in that,
i) the cell comprises on the chromosome two anti-parallelly transcribed copies of said gene of interest; and
ii) any DNA segment situated between the two copies of the gene of interest under item i) only comprises a DNA sequence which is not essential for the growth of the cell.

In a second aspect the present invention relates to a method for constructing a prokaryotic cell expressing a gene of interest comprising the construction of a cell wherein
i) the cell comprises on the chromosome two anti-parallelly transcribed copies of said gene of interest; and
ii) any DNA segment situated between the two copies of the gene of interest under item i) only comprises a DNA sequence which is not essential for the growth of the cell.

In a third aspect the invention relates to a method for production and isolation of a polypeptide of interest, comprising
i) culturing a prokaryotic cell according to the first aspect of the invention under suitable conditions permitting expression of the polypeptide of interest encoded by the gene of interest; and
ii) isolating the polypeptide of interest.

One of the advantages of a prokaryotic cell, as described herein, is that said two copies of a gene are anti-parallelly transcribed (i.e. either convergently or divergently transcribed), which minimises the risk that one copy of said gene will be lost from the cell by homologous recombination, as compared to when the genes are parallelly transcribed (see figure 1 for illustration).

This implies a further advantage of said prokaryotic cell, since it is then possible to have a stable integration of said two copies of a gene, without having a DNA segment situated between the two copies of said gene, which are essential for the growth of the cell.

Accordingly, there is no need for integrating the individual copies of the gene of interest at very distant places on the chromosome as described in EP 284126 (see figure 2 of EP 284126). This makes it relatively simpler to construct a prokaryotic cell (as described herein) as compared to the construction of a cell of EP 284126. See below for further details on preferred ways of construction of a prokaryotic cell as described herein.

Further, in the art said DNA segment essential for the growth of the cell has been an antibiotic resistance marker gene, which has been used to construct prokaryotic cells comprising more than one copy of a gene of interest (see e.g. EP 166, 628 and WO 94/14968).

Accordingly, a further advantage of a method for constructing a prokaryotic cell, as described herein, is that it provides a simple method of producing a prokaryotic cell which expresses a gene from two copies of said gene, without the cell comprising an introduced antibiotic resistance gene.

### DEFINITIONS:

Before describing the invention in details, terms of the independent aspects of the invention will be further defined below.

The term "a gene" indicates herein a gene (a DNA sequence) will is capable of being expressed into a polypeptide within said cell. Accordingly, said gene sequence will be defined as an open reading frame starting from a start codon (normally "ATG", "GTG", or "TTG") and ending at a stop codon (normally "TAA", TAG" or "TGA").

In order to express said gene there must be elements, as known in the art, in connection with the gene, necessary for expression of the gene within the cell. Such standard elements may include a promoter, a ribosomal binding site, a termination sequence, and may be others elements as known in the art.

The term "two anti-parallelly transcribed copies of said gene" denotes herein that said the genes are either convergently or divergently transcribed, i.e. present in opposite orientation relative to each other. See figure 1 for a graphic illustration.

The term "the cell comprises on the chromosome two anti-parallelly transcribed copies of said gene of interest", in connection with a prokaryotic cell according to the invention, indicates that said prokaryotic cell comprises at least said two copies of the gene of interest situated as described in the first aspect of the invention. Besides these two copies of said gene of interest said prokaryotic cell may comprises further copies of said gene on the chromosome.

Said further copies may be two further copies of said gene situated according to the invention on a distinct place on the chromosome, or may be just single copy/copies of said gene situated on another part of the chromosome.

The term "a DNA segment which only comprises a DNA sequence which is not essential for the growth of the cell" indicates a DNA segment that if it is crossed out (deleted) from the chromosome of said cell, then the cell is still capable of growing at substantially the same growth rate, under similar growth conditions, as compared to before said DNA segment was deleted.

In contrary when a DNA segment is termed "essential for growth of the cell" this indicates than if said DNA segment is deleted from the cell then said cell will NOT be capable of growing at substantially the same growth rate, under similar growth conditions, as compared to before said DNA segment was deleted.

Such an essential DNA segment, may be a part of the parental (wild-type) chromosomal sequence (as *e.g.* described in EP 284126), or may be an selectable marker gene, such as an antibiotic resistance marker gene, wherein said selectable marker may have been introduced on the chromosome (see *e.g.* EP 166628; WO 9414968).

Embodiment(s) of the present invention is described below, by way of examples only.

### DRAWINGS:

### Figure 1:

Part A illustrates two genes, situated on the chromosome of a cell, which are parallelly transcribed. As shown by crossed lines one copy of said gene may be lost from the cell by homologous recombination of the identical DNA sequences.

Part B illustrates two genes, situated on the chromosome of a cell, which are divergent transcribed. In this situation no direct identical sequences are found between the two genes, consequently, the risk of homologous recombination between the two genes, leading to the loss of one of the gene copies, is drastically minimised.

Part C illustrates two genes, situated on the chromosome of a cell, which are convergent transcribed. As for part B there is no homologous recombination between said two genes.

### Figure 2:

This figure illustrates a preferred strategy to construct a prokaryotic cell as described herein.

### Part A:

The DNA segments "ABC" and "DEF" are on the chromosome within the cell.

The DNA segments "ABC", "123", "456", "XXX" (gene of interest), and "DEF" are introduced into the cell, preferably on a temperature sensitive plasmid.

### Part B:

After homologous recombination between the DNA segments "ABC" and "DEF" the chromosome now comprises "ABC", "123", "456", "XXX", and "DEF".

A new plasmid in then transformed into the cell comprising the segments "123", "XXX", and "456", wherein the gene "XXX" is transcribed in opposite direction as compared to part A.

### Part C:

After homologous recombination between the DNA segments "123" and "456" the chromosome now comprises "ABC", "123", "XXX" "456", "XXX", and "DEF", wherein the genes of interest "XXX" are anti-parallel transcribed. In the specific example shown here they are divergently transcribed.

### Figures 3-14:

Those figures show plasmids used in working examples 1 to 3 herein to make a prokaryotic cell, according to the invention, by a method for constructing a prokaryotic cell, according to the invention.

Consequently, reference is made to examples 1 to 3, for further description of said plasmids.

### Figures 15-29:

Those figures illustrate strategies and plasmids used in working example 4 herein to make a prokaryotic cell, according to the invention, by a method for constructing a prokaryotic cell, according to the invention.

Consequently, reference is made to example 4, for further description of those figures.

### DETAILED DESCRIDTION OF THE INVENTION:

In an embodiment of the invention the gene of interest, according to the aspects of the invention described above, is a gene which is capable of expressing a polypeptide which are secreted from the cell.

Such a gene may preferably encode a fusion polypeptide comprising a signal peptide and the secreted mature polypeptide. Such signal peptides are well known in the art.

In a further embodiment of the invention, said gene encodes an enzyme. Such an enzyme may be an protease, amylase, cellulase, lipase, xylanase, phytase, and other enzymes known in the art.

In an even further embodiment of the invention, said prokaryotic cell is a gram positive prokaryotic cell, such as a *Bacillus* cell or a *Streptomyces* cell.

Preferred *Bacillus* cells are species such as *Bacillus subtilis, Bacillus licheniformis, Bacillus lentus, Bacillus alkalophilus, Bacillus amyloliquefaciens, Bacillus clausii*, *Bacillus circulans,* and a *Bacillus thuringiensis* cell.

Further, an embodiment of the invention relates to that said DNA segment, which is situated between two copies of said gene, is at least 10 bp long, more preferably from 10 bp to 6000 bp, and even more preferably from 75 bp to 4500 bp long.

An further embodiment of the invention relates to that said DNA segment, which is situated between two copies of said gene, further does not comprise a gene encoding a screenable protein, such as a Green Fluorecent protein (GFP). Such a screenable protein may be used to construct a prokaryotic cell comprising more than one copy of a gene of interest (see DK 0792/97; and WO 99/01562 for a further description of such screenable proteins).

An even further embodiment of the invention relates to a prokaryotic cell of the first aspect of the invention or a method of the second aspect of the invention, wherein said DNA segment, which is situated between the two copies of the gene of interest, does not comprise an antibiotic resistance marker gene, such as an ampicillin resistance gene; an erythromycin resistance gene; a kanamycin resistance gene; a neomycin resistance gene; or a chloramphenicol resistance gene.

See e.g. working example 3 and 4 for examples of such antibiotic resistance marker gene free cells of the invention.

Said method according to the second aspect of the invention and its embodiments described herein may be performed according to any of the in the art known techniques for introducing DNA segments/fragments into the chromosome of a prokaryotic cell, in particular any of the known techniques for introducing said DNA segments into the chromosome by homologous recombination. A preferred strategy is to transform a cell with a plasmid comprising said DNA segment of interest and a temperature sensitive origin of replication. The cell in then cultured a the non-permissive temperature for replication of said plasmid and the plasmid recombine with the chromosome within the cell by homologous recombination.

For further description of such methods reference is made to EP 0 284 126; EP 166 628; WO 94/14968; Maniatis, T., Fritsch, E. F., Sambrook, J. "Molecular Cloning. A laboratory manual". Cold Spring Harbor Laboratories, 1982; Ausubel, F. M., et al. (eds.) "Current Protocols in Molecular Biology". John Wiley and Sons, 1995; Harwood, C. R., and Cutting, S. M. (eds.) "Molecular Biological Methods for Bacillus".

General strategies for performing said method according to the second aspect of the invention and its embodiments described herein are disclosed in working examples herein (see example 3 and 4).

A preferred strategy, based on homologous recombination, is shown in figure 2 herein. See working example 4 for an practical example of this strategy.

An advantage of a method as described in a working example herein and as described in figure 2, may be that the two copies of the gene of interest are inserted by a precise mechanism into the same locus in the host chromosome. This is preferable if a particularly well-suited locus for such a gene is known.

Accordingly, an embodiment of method for construction a prokaryotic cell expressing a gene, of the invention, is wherein the two copies of the gene of interest are inserted by a precise mechanism into the same locus in the host chromosome.

The specific culturing conditions under step i) and the specific isolation protocol of the polypeptide of interest under step ii) of the method for production and isolation of a polypeptide of interest according to the third aspect of the invention may be performed according to any standard protocol known to the skilled person.

As stated above, an advantage of a prokaryotic cell as described herein is that it is stable in the sense that it during fermentation is able to maintain the two copies of said gene on the chromosome.

Accordingly, it is specially suitable for large scale industrial fermentation's.

Such large scale industrial fermentation's may be characterised by that a) the polypeptide of interest is produced at a relatively high yield or b) that a large scale fermentation process is used.

Consequently, an embodiment of the method for production and isolation of a polypeptide of interest, of the third aspect the invention, is wherein the culturing of a prokaryotic cell of item i) is done under conditions wherein the polypeptide of interest is expresses in an amount of at least 2 g polypeptide (dry matter) / kg culture medium; preferably in an amount of at least 3 g polypeptide (dry matter) / kg culture medium; and most preferably in an amount of at least 5 g polypeptide (dry matter) / kg culture medium.

Further, an embodiment of the method for production and isolation of a polypeptide of interest, of the third aspect the invention, is wherein the culturing of a prokaryotic cell of item i) is in a fermentation process on a volume scale which is > 10 m³; preferably > 25 m³; more preferably > 50 m³; and most preferably > 100 m³.

### MATERIALS AND METHODS

In vitro DNA work, transformation of bacterial strains etc. were performed using standard methods of molecular biology (Maniatis, T., Fritsch, E. F., Sambrook, J. "Molecular Cloning. A laboratory manual". Cold Spring Harbor Laboratories, 1982; Ausubel, F. M., et al. (eds.) "Current Protocols in Molecular Biology". John Wiley and Sons, 1995; Harwood, C. R., and Cutting, S. M. (eds.) "Molecular Biological Methods for Bacillus". John Wiley and Sons, 1990).
If not otherwise mentioned enzymes for DNA manipulations were used according to the specifications of the suppliers.

Media used (TY, BPX and LB agar) have been described in EP 0 506 780. LBPSG agar is LB agar supplemented with phosphate (0.01 M K₃PO₄), glucose (0.4 %), and starch (0.5 %).

### Example 1. Deletion of the α-amylase (amyL) promoter from the B. licheniformis chromosome.

### 1A. Plasmid constructions:

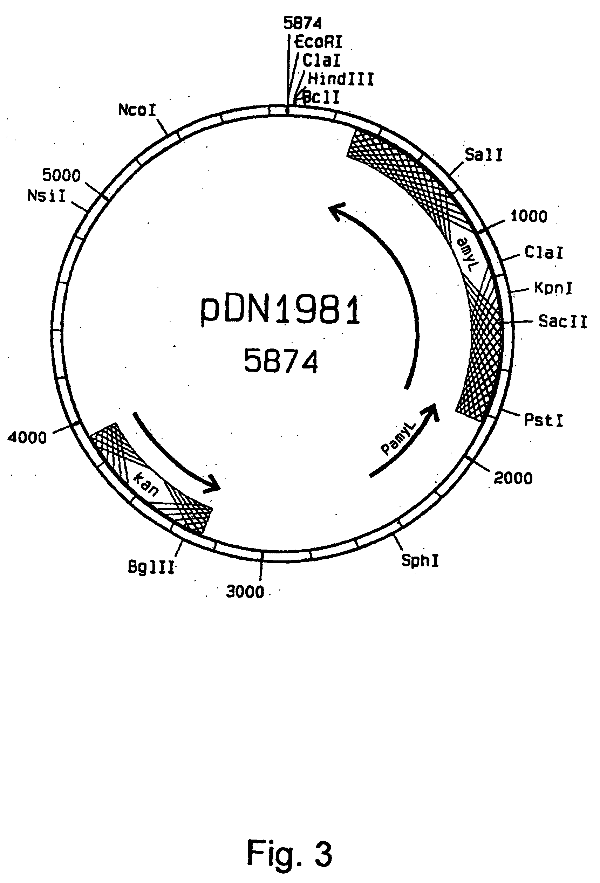

The α-amylase (*amyL*) gene from *B. licheniformis* was cloned on a 2.4 kb SphI-HindIII fragment resulting in plasmid pDN1981 (Fig. 3), as described by Jørgensen et al., 1990. "kan" denotes the pUB110 derived kanamycin resistance gene, and "PamyL" denotes the alpha-amylase promoter region.
(Jørgensen, P. L., Hansen, C. K., Poulsen, G. B., Diderichsen, B. (1990). In vivo genetic engineering: homologous recombination as a tool for plasmid construction. Gene 96, 37-41.)

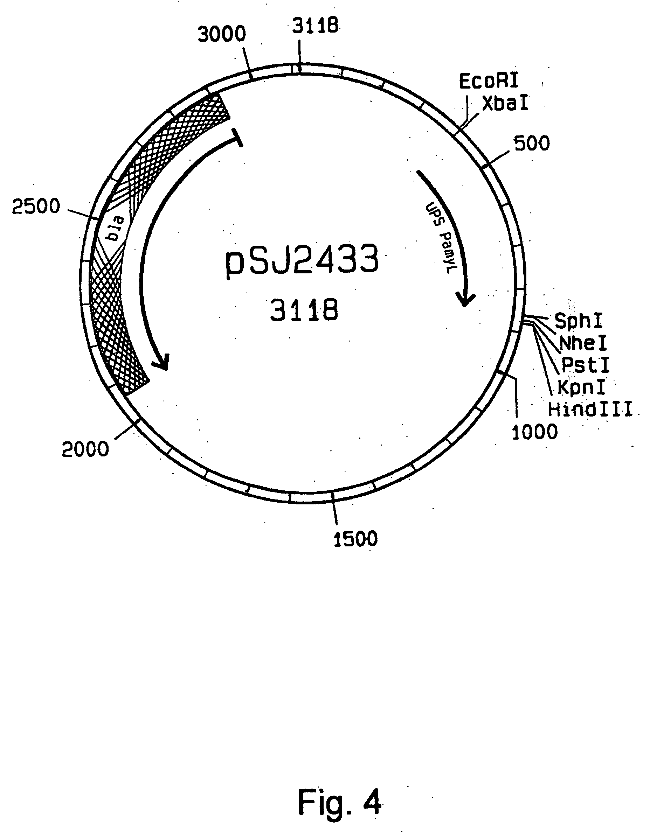

pSJ2433 (Fig. 4) contains the first 450 basepairs of this sequence, denoted UPS_P*amyL*, cloned into pUC19 (Yanish-Perron et al., 1985 (Yanish-Perron, C., Vieira, J., Messing, J. (1985). Improved M13 phage cloning vectors and host strains: nucleotide sequences of the M13 mp18 and pUC19 vectors. Gene 33, 103-119)), and was constructed as follows: pDN1981 DNA was used as template for PCR amplification with primers LWN4739 + LWN4740, the resulting 0.5 kb fragment digested with EcoRI and HindIII, ligated to EcoRI+HindIII digested pUC19, and transformed into *E. coli* SJ2 (Diderichsen et al., 1990 (Diderichsen, B., Wedsted, U., Hedegaard, L., Jensen, B. R., Sjøholm, C. (1990). Cloning of *aldB*, which encodes α-acetolactate decarboxylase, an exoenzyme from *Bacillus brevis*)) by electroporation selecting ampicillin resistance (AmpR), 200 µg/ml. "bla" denotes the pUC19 ampicillin resistance gene.
Two transformants were kept as SJ2433 and SJ2434. Identity of the insert was confirmed by DNA sequencing of the ends, but the entire insert was not sequenced.

LWN4739
HindIII KpnI PstI NheI SphI <---- amyLV2 464-437-
5'-TGAGTAAGCTTGGTACCCTGCAGGCTAGCGCATGCGCTGAGATACAGTTACCAATT-

------>
GATAGCC-3'

LWN4740
EcoRI XbaI <---- amyLV2 18-43 ------>
5'-TGAGTGAATTCTCTAGACCTTCTTTGTGCTTGGAAGCAGAGCC-3'

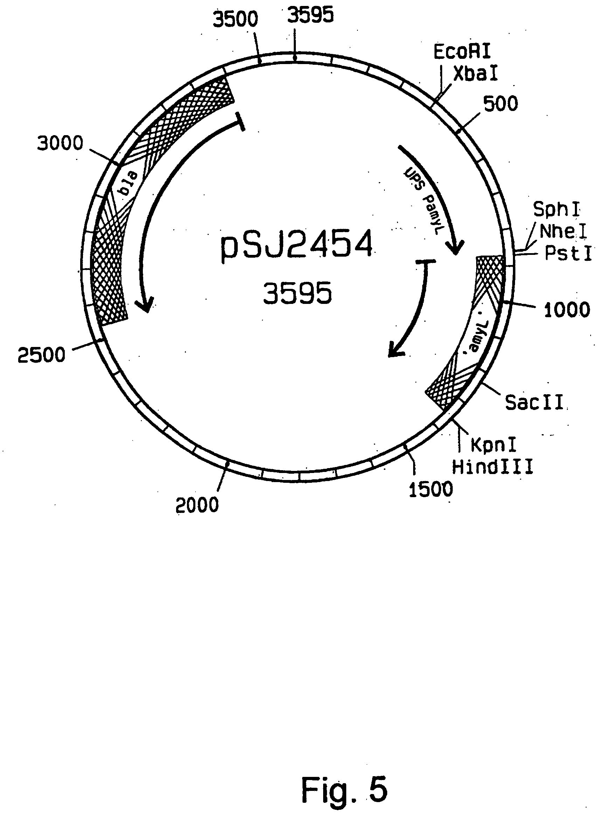

pSJ2454 (Fig. 5) was derived from pSJ2433 by ligation of a 0.5 kb PstI-KpnI fragment from the α-amylase (*amyL*) gene on pDN1981 (denoted '*amyL*') to PstI+KpnI digested pSJ2433 and transformation of *E*. *coli* SJ6 (Diderichsen et al., 1990) by electroporation, selecting AmpR, 200 µg/ml.

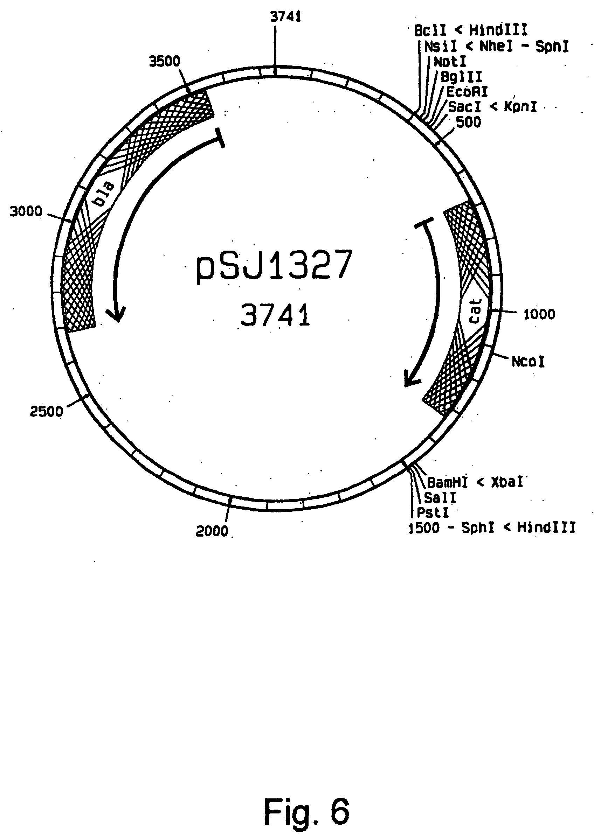

pSJ1327 (Fig. 6) contains a pC194 (Horinouchi and Weisblum, 1982. (Horinouchi, S., and Weisblum, B. (1982). Nucleotide sequence and functional map of pC194, a plasmid that specifies chloramphenicol resistance. J. Bacteriol., 173, 559-567)) derived *cat* gene inserted into a polylinker in a pUC19 derivative. The *cat* gene, which specifies chloramphenicol resistance, was excised from pDN1600 as a 1.0 kb BamHI-BglII fragment, ligated to BamHI digested pDN3000 (Diderichsen et al., 1990), and transformed into *E. coli* SJ6 selecting AmpR (200 µg/ml).

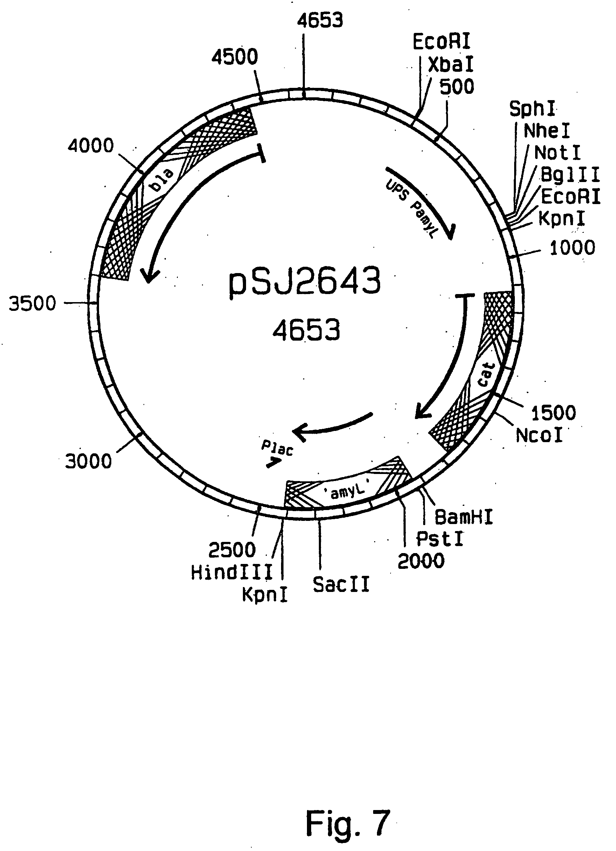

pSJ2643 (Fig. 7) contains the *cat* gene inserted between the two *amyL* derived segments in pSJ2454. The *cat* gene was excised as a 1.1 kb NheI-XbaI fragment from pSJ1327, ligated to NheI digested pSJ2454, and transformed to *E. coli* SJ6 by electroporation, selecting chloramphenicol resistance (CamR, 6 µg/ml), to create pSJ2643. "Plac" indicates the pUC19 derived beta-galactosidase promoter.

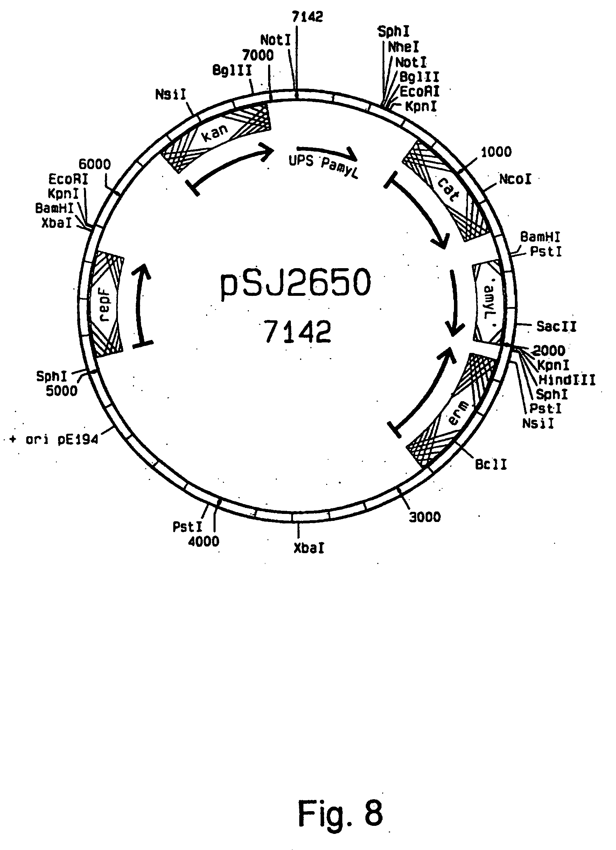

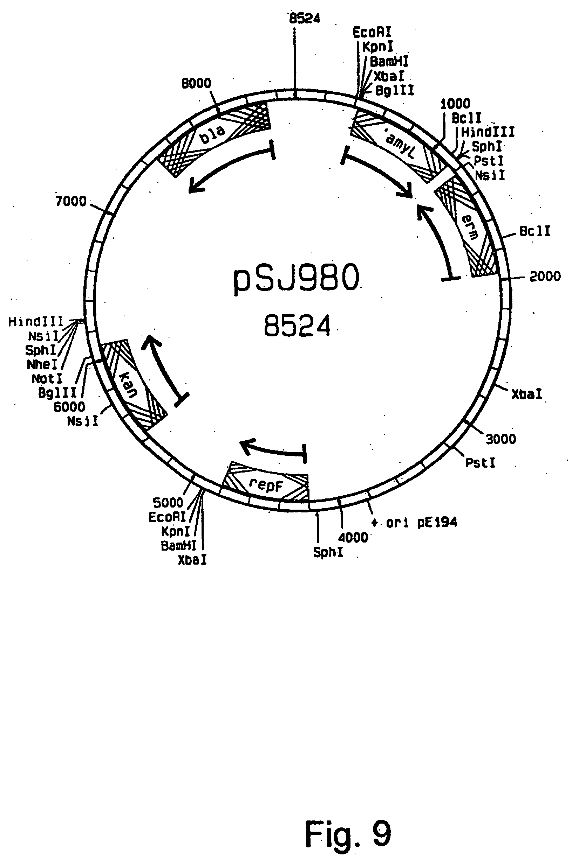

pSJ2650 (Fig. 8) contains the UPS_P*amyL-cat*-'*amyL*' segment on a temperature-sensitive *Bacillus* plasmid. pSJ2643 was digested with XbaI + HindIII, the 2.0 kb fragment isolated, ligated to the 5.1 kb NheI-HindIII fragment from pSJ980 (Fig. 9; United States Patent 5,698,415 ), and transformed into *B. subtilis* DN1885 (Diderichsen et al., 1990) competent cells selecting CamR and kanamycin resistance (KanR), 6 µg /ml and 10 µg /ml, respectively, on LBPSG plates (WO 96/23073)), to give pSJ2650. "repF" indicates the pE194-derived replication protein gene, "+ ori pE194" indicates the pE194 replication origin, and "erm" indicates the erythromycin resistance gene of pE194. This may also be denoted "ermC".

### 1B. B. licheniformis transformation:

The *B. licheniformis* strain described in example 6 of United States Patent 5,698,415, which contains one chromosomal copy of the α-amylase (*amyL*) gene expressed from a mutant *amyL* promoter, was used as host strain. pSJ2650 was introduced into this strain by protoplast transformation (Akamatzu and Sekiguchi, 1984 (Akamatsu, T., Sekiguchi, J. (1984). An improved method of protoplast regeneration for Bacillus species and its application to protoplast fusion and transformation. Agric.Biol. Chem., 48, 651-655)), selecting for erythromycin resistance (ErmR, 2 µg/ml) at 30 °C. Regenerants were isolated after two weeks incubation. Three strains were kept, SJ3034, SJ3035, and SJ3036.

### 1C. Chromosomal integration and excision:

Strains SJ3035 and SJ3036 were streaked on plates with 6 µg /ml chloramphenicol and 5 µg /ml erythromycin, and incubated at 50 °C. 8 amylase-negative colonies from each strain were checked by PCR amplification using primers LWN4726 + LWN3825, and all gave an amplified fragment of the correct size. The amplified fragment extends from the NcoI site within the *cat* gene to a position in the *amyL* gene 157 bp downstream from the KpnI site, 1007 basepairs in total. This fragment will only arise if pSJ2650 has integrated via the '*amyL*' homology.

LWN3825
<-amyLV2 1341-1322->
5'-CTGCTGCGACATCAGGATGG-3'

LWN4726
<-pDN3060 548-528-->
5'-CATGGACTTCATTTACTGGG-3'

Each colony was taken through two consecutive overnight incubations in 10 ml TY medium (WO 91/09129) without antibiotics at 30 °C, plated on plates containing 6 µg/ml chloramphenicol, replica plated after overnight incubation at 30 °C to plates containing 6 µg/ml chloramphenicol plus/minus 5 µg/ml erythromycin, and putative erythromycin sensitive colonies restreaked on the same type of plates.
Three chloramphenicol resistant, erythromycin sensitive strains were isolated:
SJ3047 from SJ3035, colony 1.
SJ3048 from SJ3035, colony 2.
SJ3049 from SJ3036, colony 1.

Integration of the cat gene into the *amyL* promoter region was confirmed by southern analysis.
A restriction map of the *amyL* gene region had previously been constructed, revealing the *amyL* promoter to be situated on a 3.35 kb HindIII fragment and on a 1.9 kb ClaI fragment.

The replacement of the amyL promoter with the cat gene should give a net insertion of 835 basepairs. No additional HindIII or ClaI sites are inserted with the *cat* gene.
Consequently, chromosomal DNA was extracted from SJ3047-49, digested separately with HindIII and ClaI, separated on agarose gels, transferred by vacuum blotting to immobilon-N membranes, and probed with pSJ1325 (a pUC19 plasmid containing the cat gene) ³²P-labelled by nick-translation.

Hybridization was to a 4.2 kb HindIII fragment in SJ3047 and SJ3048, as expected, but to a somewhat larger fragment in SJ3-049. Hybridization was to a 2.7 kb ClaI fragment in SJ3047 and SJ3048, again as expected.

Consequently, strains SJ3047 and SJ3048 which are amylase-negative have the *amyL* promoter replaced by a *cat* gene, as desired.

### Example 2. Isolation of an improved α-amylase (amyL) promoter.

### 2A. Plasmid constructions.

United States Patent 5,698,415 claims variant promoters derived from the *B. licheniformis amyL* promoter. With reference to claim 1 in the above patent, such a variant promoter is a fragment of the sequence given in this claim, in which N²-N⁹ has the sequence ATGTATCA. Such a variant promoter was constructed by incorporating the desired mutation into a long PCR primer (#28902) covering the *amyL* promoter region. Another PCR primer, LWN3216, reads upstream from a position spanning the PstI site:in the AmyL signal peptide coding region. Together, these primers allow PCR amplification of a variant *amyL* promoter fragment.

#28902:
XbaI SphI ←---- fragment of sequence
5'-GCTCTAGAGCATGCTGGAAGAAAATATAGGGAAAATGGTA-
given in claim 1, US Patent 5,698,415
CTTGTTAAAAATTCGGAATATTTATACAATATCATATGTATCAC-
-------→
ATTGAAAGGGGAGGAGAATCATG-3'

LWN3216:
5'-GCTGCTGCAGAATGAGGCAG-3'

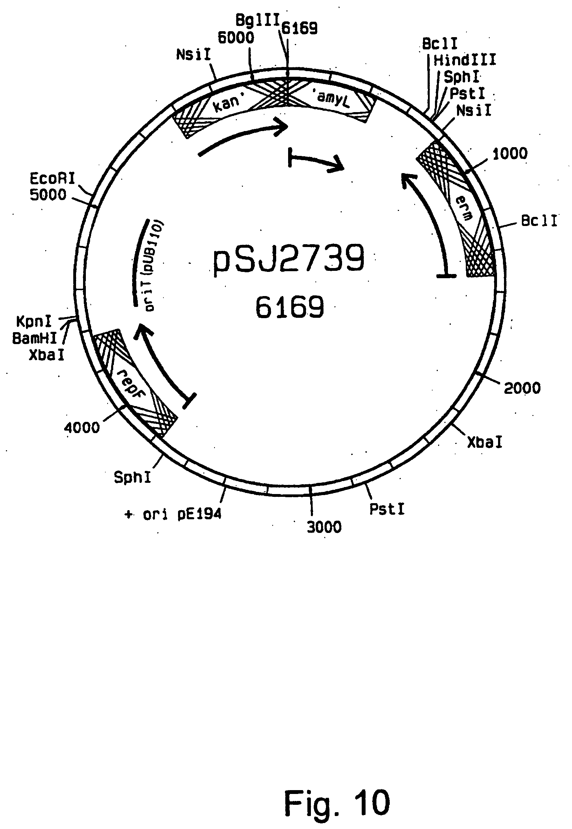

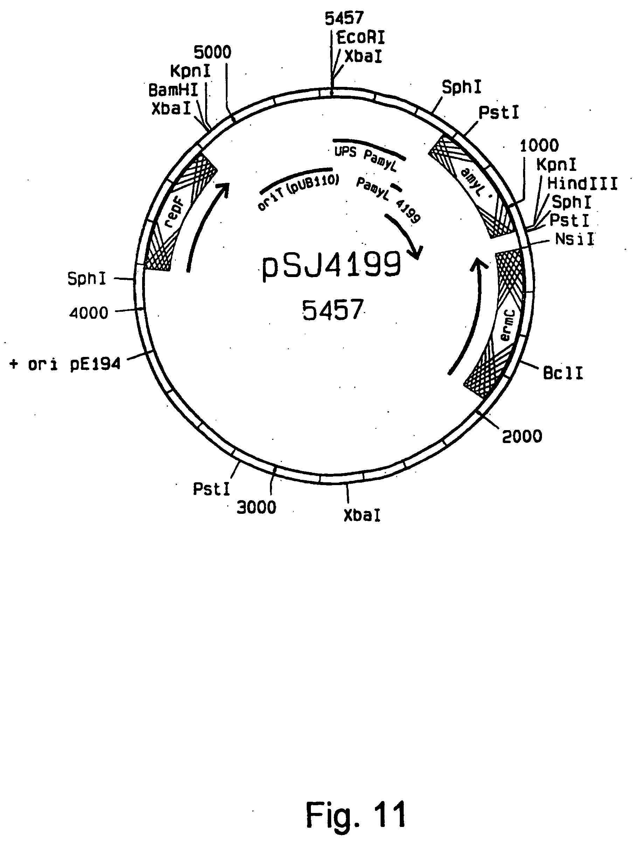

A mobilizable, temperature-sensitive plasmid containing the variant promoter flanked by *amyL* DNA in a form suitable for integration into the chromosome of strain *B. licheniformis* SJ3047 was constructed as follows:
The substrate for PCR amplifications was pDN1981, which had been digested with BglII. Primer #28902 was used together with primer LWN3216, at an annealing temperature of 45 °C. A correctly sized PCR product was obtained, digested with SphI+PstI, ligated to the 3.6 kb SphI-PstI fragment of pSJ2643, the ligation mixture then digested with EcoRI+HindIII, and the 1.1 kb fragment (isolated from a mixture of fragments) ligated to the 4.4 kb EcoRI-HindIII fragment from pSJ2739 (Fig. 10; described in WO 96/23073 "DNA integration by transposition"). The ligation mixture was transformed into competent *B. subtilis* DN1885 selecting erythromycin resistance (5 µg/ml) at 30 °C. The recombinant plasmids were analyzed by restriction mapping, and the *amyL* promoter region on the plasmid designated pSJ4199 (Fig. 11) was DNA sequenced, using primer LWN3207, and found to have the desired sequence. "oriT(pUB110)" denotes the cis acting sequence of pUB110 necessary for conjugative mobilization of the plasmid, see WO 96/23073. "PamyL 4199" denotes the variant *amyL* promoter.

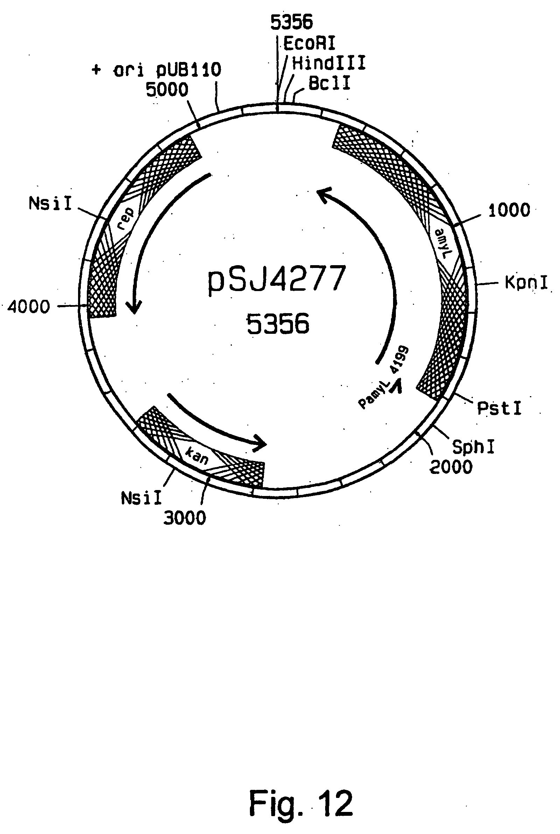

The variant *amyL* promoter was used to replace the *amyL* promoter found on plasmid pDN1981. It was excised as a 0.2 kb SphI-PstI fragment from pSJ4199, and ligated to the 5.0 kb SphI-PstI fragment from pDN1981. The ligation mixture was transformed into *B*. *subtilis* DN1885, selecting kanamycin resistance (10 µg/ml). Two transformants were kept, as SJ4277 (DN1885/pSJ4277; fig. 12) and SJ4278 (DN1885/pSJ4278). "rep" denotes the pUB110 replication protein gene.

### 2B. Mobilization into B. licheniformis and chromosomal integration.

Plasmid pSJ4199 was transformed into competent cells of *B. subtilis* PP289-5 (dal-, pLS20, pBC16; WO 96/23073, example 4) selecting erythromycin (5 µg/ml) and tetracycline (5 µg/ml) resistance on D-alanine (100 µg/ml) plates at 30 °C.
Two transformants were kept, SJ4237 and SJ4238..

Each of these donor strains were used to transfer their plasmid into *B. licheniformis* by conjugation, essentially as described in WO 96/23073, example 11. Transconjugants were almost exclusively tetracycline sensitive.
One transconjugant derived from each donor strain was kept:
SJ4258 (from SJ4237) and SJ4259 (from SJ4238), both containing pSJ4199.

These strains were streaked on LBPSG plates supplemented with chloramphenicol (6 µg/ml) and erythromycin (5 µg/ml) at 50 °C overnight. Amylase positive colonies were obtained from each strain, and 4 such colonies from each strain were inoculated in TY medium without antibiotics at 30 °C for 3-4 subsequent transfers, to allow replication, excision, and loss of the plasmid. Amylase positive, erythromycin sensitive colonies were obtained from each transconjugant strain, and kept as SJ4270 (from SJ4258) and SJ4271 (from SJ4259).

### 2C. Test of variant promoter strain.

The efficiency of the variant promoter was tested in shake flask experiments, in which α-amylase production from each of the integrant strains was compared to α-amylase production from the control strain (the strain, from which SJ3047 was derived by deletion of the promoter). Incubation was in duplicate in BPX medium (EP 0 506 780), for 7 days at 37 °C, and α-amylase activity measured at day 2, 5 and 7.

The activity is given relative to the highest activity measured from the control strain.

| **Strain** | **Day 2** | **Day 5** | **Day 7** |
|---|---|---|---|
| | yield | yield | yield |
| **control (1)** | 8.5 | 57 | 90.5 |
| **control (2)** | 9.5 | 65 | 100 |
| **SJ4270 (1)** | 13.5 | 106.5 | 170.5 |
| **SJ4270 (2)** | 14 | 89.5 | 155 |
| **SJ4271 (1)** | 12.5 | 90.5 | 174 |
| **SJ4271 (2)** | 14 | 89.5 | 170 |

The variant promoter present in strains SJ4270 and SJ4271 is clearly improved with respect to α-amylase production, relative to the promoter of the control strain.

### Example 3: Construction of a strain containing two divergently transcribed expression cassettes.

### 3A. Plasmid constructions:

A plasmid was designed, in which an entire copy of the *amyL* gene was inserted between the *UPS_PamyL* segment and the *PamyL*_*4199* promoter on pSJ4199, so that the two *amyL* promoters on the resulting plasmid would read in opposite directions.

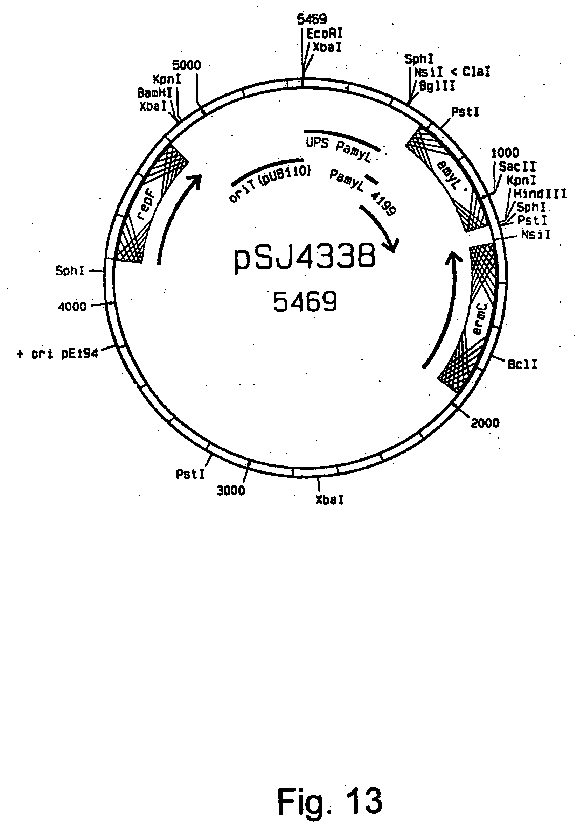

Plasmids pSJ4338 (Fig. 13) and pSJ4339 were constructed. These are almost identical to the integration plasmid pSJ4199, except for a few extra restriction sites introduced between the *UPS_PamyL* and *PamyL_4199* segments. Plasmid pSJ4278 was used as template in a PCR amplification with primers #113123 and LWN3216.

#113123:
SphI ClaI BglII
5'-GTCAGCATGCATCGATAGATCTTGGAAGAAAATATAGGG-3'

LWN3216:
5'-GCTGCTGCAGAATGAGGCAG-3'

An amplified fragment of 0.19 kb was obtained, purified, 'and digested with SphI + PstI. It was ligated to the 3.5 kb SphI-PstI fragment of pSJ2643, the ligation mixture digested with EcoRI + HindIII, the fragment of 1.13 kb purified, and ligated to EcoRI + HindIII digested pSJ2739. This ligation mixture was then transformed into competent DN1885 selecting erythromycin resistance (5 µg/ml) at 30 °C. Three transformants were obtained. Strain SJ 4338 (DN1885/pSJ4338) contained a plasmid, which was correct as seen by restriction analysis, and the *amyL* promoter region was found to be correct by DNA sequencing.

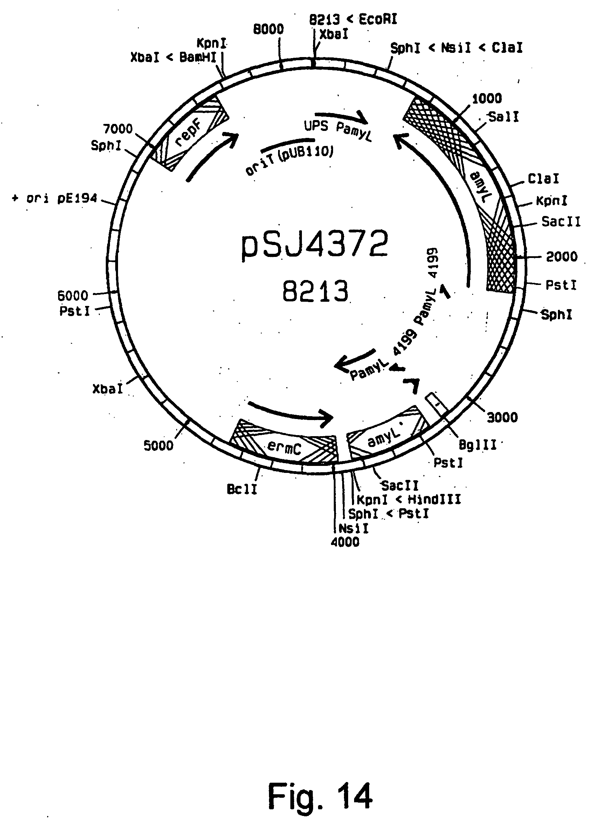

Plasmids pSJ4372 and pSJ4373 (Fig. 14) were then constructed by insertion of the entire *amyL* gene into the above integration plasmid. Thus, pSJ4277 was digested with BclI + BglII (+ with NcoI, to further digest the unwanted fragment) and the 2.8 kb fragment purified. This was ligated to BglII digested pSJ4338, and the ligation mixture transformed into competent DN1885 selecting erythromycin resistance (5 µg/ml) at 30 °C. Amylase positive transformants, deemed correct by restriction analysis, were SJ4372 (DN1885/pSJ4372) and SJ4373 (DN1885/pSJ4373).

### 3B. Transfer to B. licheniformis and chromosomal integration:

These plasmids were subsequently transformed into conjugative donor strain host PP289-5, resulting in strains SJ4378 and SJ4379 (both PP289-5/pSJ4373; Erm^{R} Tet^{R} Dal⁻).
Plasmid pSJ4373 was transferred into *B. licheniformis* SJ3047 by conjugation as previously described.
Transconjugants (4 using SJ4378 donor, 9 using SJ4379 donor) appeared after two days.
4 strains were kept: SJ4395 and SJ4396 from SJ4378 donor, SJ4397 and SJ4398 from SJ4379 donor.

The strains were streaked on LBPSG plates with erythromycin (5 µg/ml) at 50 °C, and 10 single colonies from each strain then inoculated into 10 ml TY cultures and propagated at 30 °C overnight. Cultures were then spread to single colonies on LBPSG plates, these plates incubated overnight at 30 °C, replica plated to LBPSG plates with erythromycin (5 µg/ml), and erythromycin resistance and amylase phenotype scored after overnight incubation. Most colonies were erythromycin sensitive, but amylase negative. From one culture, however, was an erythromycin sensitive, amylase positive strain isolated. This was kept as SJ4414.

### 3C. Test of two-copy strain:

The performance, in BPX shake flasks, of two-copy strain SJ4414 was compared to that of SJ4270 (the corresponding one-copy strain) in two separate experiments.
BPX shake flasks were incubated for 7 days at 37 °C, no antibiotics added, and α-amylase activity measured. For each experiment, activities given are relative to the activity obtained from the one-copy strain.

| **Experiment A** | | |
|---|---|---|
| Strain | Yield | pH of spent broth |
| SJ4270 | 100 | 8.0 |
| SJ4414 | 153 | 7.5 |

| **Experiment B** | | |
|---|---|---|
| Strain | Yield | pH of spent broth |
| SJ4270 (1) | 100 | 8.5 |
| SJ4414 (1) | 126 | 8.0 |

Experiments A and B clearly demonstrate that the **two-copy strain SJ4414** gave a **higher yield** as compared to the corresponding one-copy strain SJ4270.
Further, SJ4414 samples were spread on LBPSG. All single colonies (about 30, from each flask, including those with low pH) were amylase-positive, **indicating the stable maintenance of the** two integrated copies.

### Example 4: Chromosomal gene insertion in two successive steps.

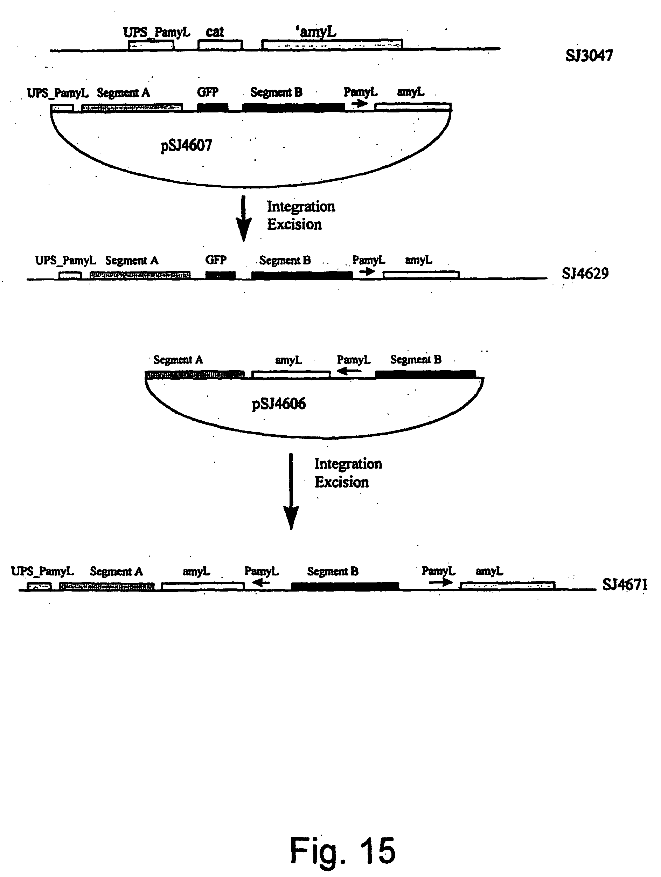

As an alternative to the construction pathway described above (example 3) a temperature sensitive, mobilizable vector pair was designed and constructed to allow for easy cloning and integration of any expression cassette. The first vector contains two regions with homology to neighbouring segments of the host cell chromosome. In between, it contains a multilinker site and a large (e.g. 5 kb) segment of "neutral" DNA. The second vector contains only the large segment of "neutral" DNA, but has the multilinker site inserted in the middle of this segment, and in the opposite relative orientation as compared to the first vector. The vectors and the strategy behind their use is illustrated in the schematic drawing in figure 15. In addition to the previously defined notation, "segment A" denotes one half of the large "neutral" DNA segment, "segment b" denotes the other half of this segment, and "GFP" denotes a gene expressing Green Fluorescent Protein.

As "neutral" DNA suitable for incorporation into production strains was chosen a composite of two segments from the *Bacillus subtilis* 168 pps gene region (Accession Number Z34883 in Gen-Bank/EMBL), taken from strain SJ2692 which is the *B. subtilis* strain used in the international genome sequencing programme . One segment is entirely internal in gene *pps2,* the other segment entirely internal in *pps4*. They were chosen because they contained very few restriction enzyme sites, and because they, as cloned, would not be expected to encode any gene products.

### 4A. Construction of vector for integration of first expression cassette copy:

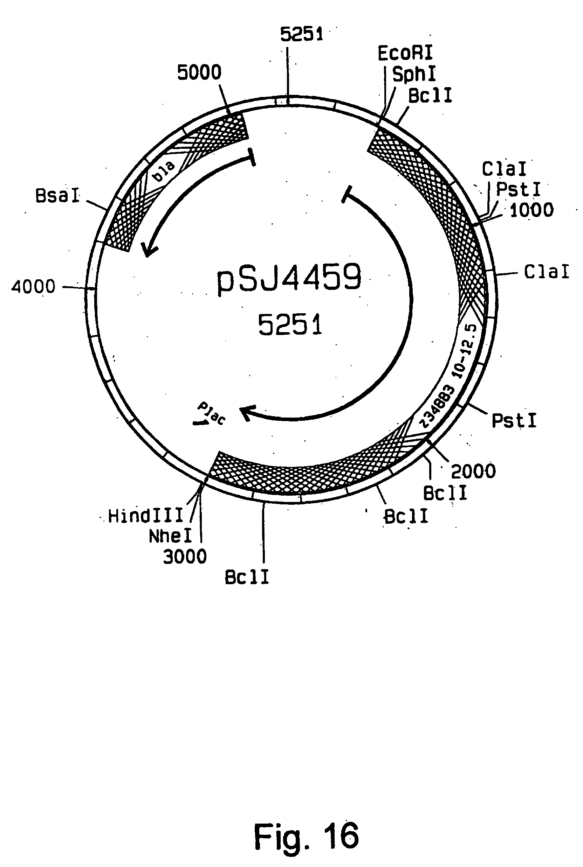

Plasmid pSJ4459 (figure 16) was constructed as follows: Chromosomal DNA from SJ2692 was FOR amplified with primers #119882 and # 119883.

#119882:
EcoRI SphI← z34883 9964-9987----→
5'-CAGTGAATTCGCATGCAGCAGGTAGTTCTATCAAACCG-3'

#119883:
HindIII NheI← z34883 12565-12540--→
5'-GACTAAGCTTGCTAGCCGCTGGATGTTAATGGCATCTGGC-3'

The amplified 2.6 kb fragment was digested with EcoRI and HindIII, and ligated to EcoRI + HindIII digested pUC19. The ligation mixture was transformed, by electroporation, into *E. coli* SJ2 selecting ampicillin resistance (200 µg/ml) on IPTG X-gal plates. Four white colonies were picked for plasmid preparation. All were correct, and could be digested with enzymes EcoRI, HindIII, SphI and NheI. One strain was kept as SJ4459 (SJ2/pSJ4459). "z34883 10-12.5" denotes the amplified fragment from *pps*2.

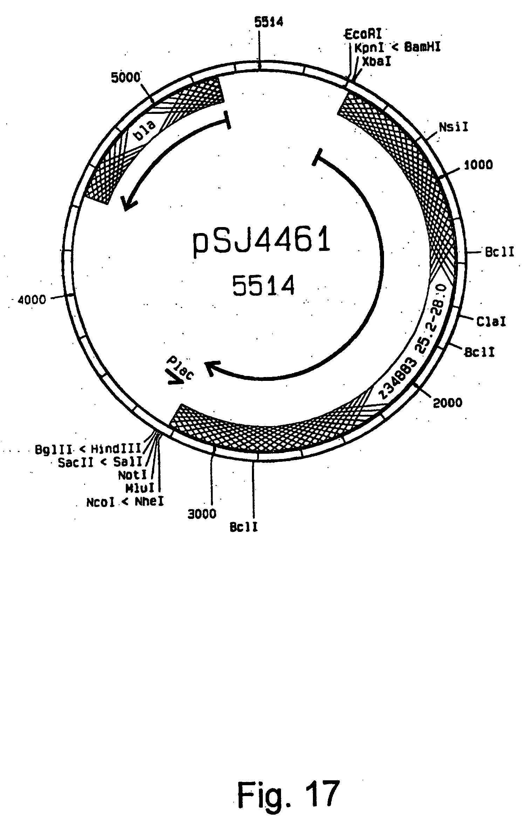

Plasmid pSJ4461 (figure 17) was constructed as follows: Chromosomal DNA from SJ2692 was PCR amplified with primers #119884 and #119885.

#119884:
XbaI ← z34883 25234-25260 ----→
5'-CAGTTCTAGACTTTTACAATAGAAGGAAAAGTCACCC-5'

#119885:
HindIII BglII SalI SacII NotI MluI NheI NcoI
5'-GACTAAGCTTAGATCTGAGCTCCGCGGCCGCACGCGTGCTAGCCATGG

<- z34883 28038-28015 →
-CCTCTAACAGATTTCGAGGGCAG-3'

The amplified 2.8 kb fragment was digested with EcoRI and HindIII, and ligated to EcoRI + HindIII digested pUC19. The ligation mixture was transformed, by electroporation, into *E. coli* SJ2 selecting ampicillin resistance (200 µg/ml) on IPTG X-gal plates. Four white colonies were picked for plasmid preparation. Three were correct, and could be digested with enzymes HindIII, XbaI, BglII, SalI, SacII, NotI, MluI, NheI, NcoI. One transformant was kept as SJ4461 (SJ2/pSJ4461). "z34883 25.2-28.0" denotes the amplified fragment from *pps*4.

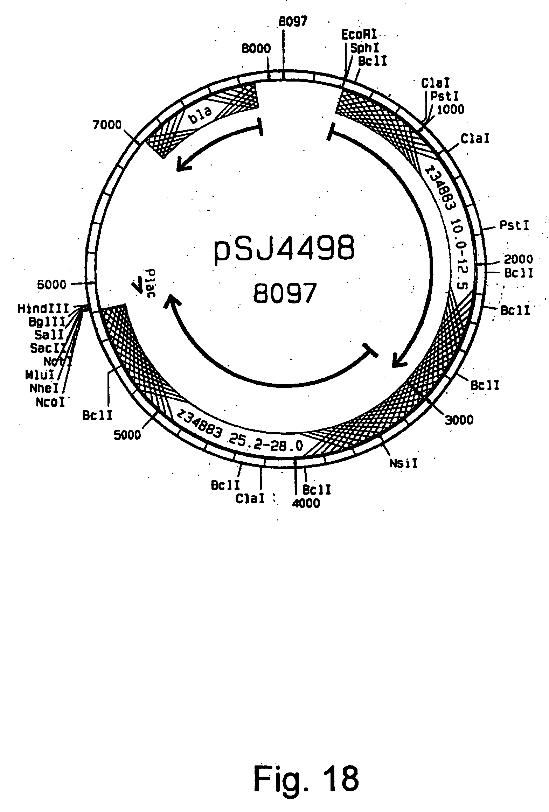

Plasmid pSJ4498 (figure 18) was constructed as follows:
Plasmid pSJ4459 was digested with EcoRI+NheI (+ with BsaI to further digest the unwanted part) and the 2.6 kb EcoRI-NheI fragment purified from an agarose gel. The fragment was ligated to EcoRI + XbaI digested pSJ4461, and the ligation mixture transformed (electroporation) into *E. coli* SJ6 selecting ampicillin resistance (6 µg/ml). One strain was kept as SJ4498 (SJ6/pSJ4498).

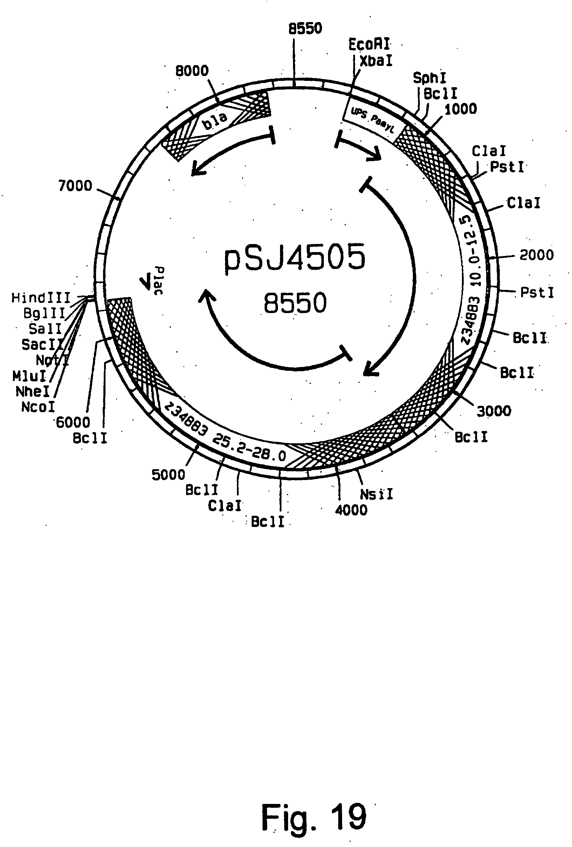

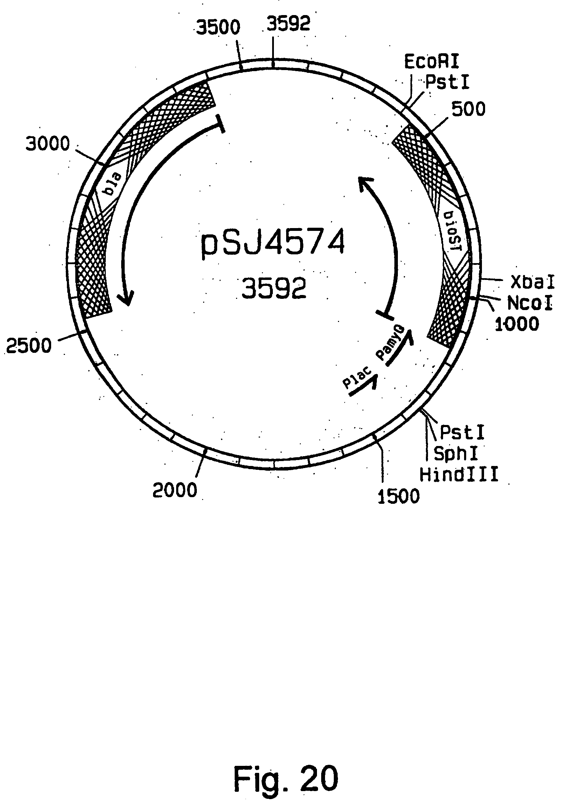

Plasmid pSJ4505 (figure 19) was constructed as follows:
pSJ4498 was digested with SphI and HindIII, and the 5.4 kb fragment gel purified. This was ligated to the 3.2 kb SphI-HindIII. fragment purified from pSJ2643 (described above, figure 7), and the ligation mixture transformed (electroporation) into *E. coli* SJ6 selecting ampicillin resistance (6 µg/ml). One transformant was kept as SJ4505 (SJ6/pSJ4505).

In order to allow visual screening for insertion of the second gene copy, a gene encoding green fluorescent protein was inserted between the two pps gene segments in the first integration vector. The first insertion of the gene of interest using this system would then render the cells GFP positive, whereas the correct insertion of the second copy of the gene of interest would render the cells GFP negative again. A plasmid expressing green fluorescent protein is pSJ4574 (figure 20), described in EXAMPLE 1 and Fig. 3 in patent application DK 0792/97; and WO 99/01562. "bioST" denotes the GFP gene, and "PamyQ" the promoter from the *Bacillus amyloliquefaciens* alpha-amylase gene.

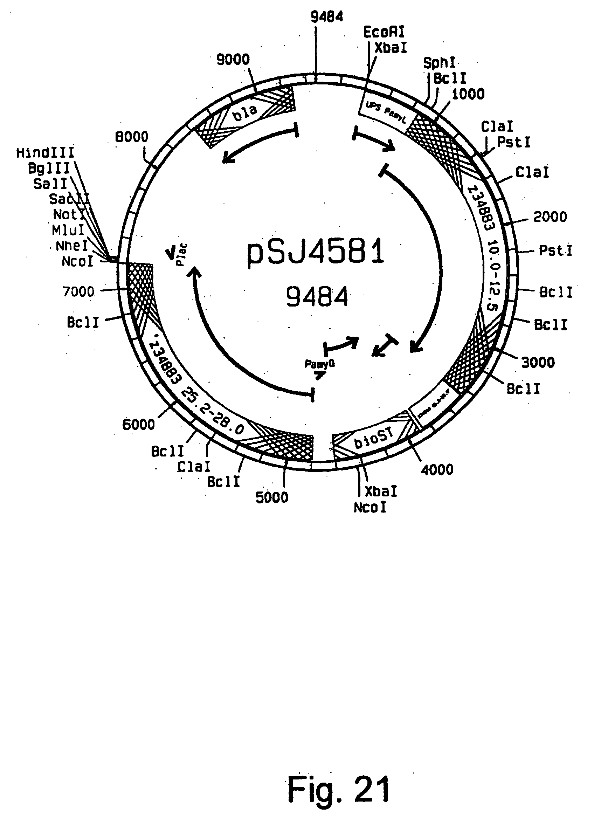

Plasmid pSJ4581 (figure 21) was constructed as follows:
pSJ4574 was digested with PstI, and the 0.95 kb fragment gel purified. This fragment was ligated to NsiI digested pSJ4505, and the ligation mixture transformed (electroporation) into E. coli SJ2 selecting ampicilin resistance (200 µg/ml). One transformant was kept as SJ4581 (SJ2/pSJ4581).

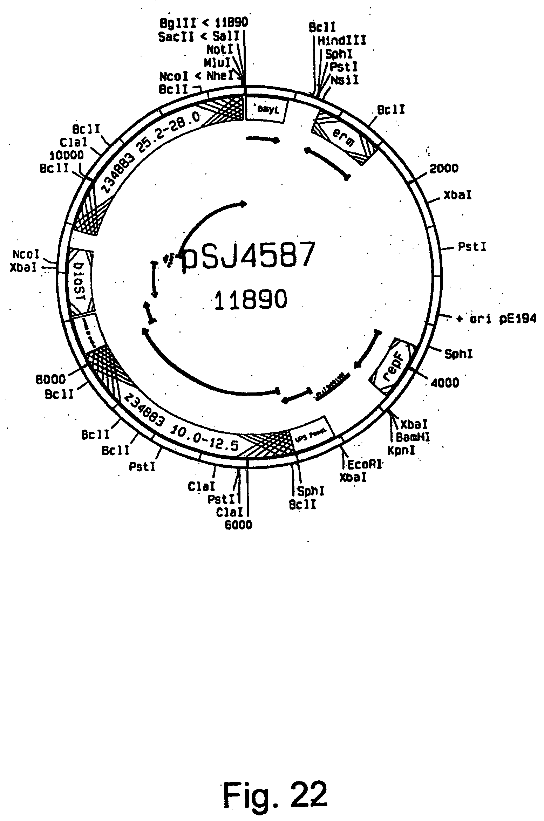

Plasmid pSJ4587 (figure 22) was constructed as follows:
pSJ4581 was digested with EcoRI + BglII, and the 6.9 kb fragment gel purified. This fragment was ligated to the 5.0 kb EcoRI-BglII fragment from pSJ2739 (described above, figure 10), and the ligation mixture was transformed into B. subtilis DN1885 competent cells selecting erythromycin resistance (5 µg/ml) at 30 °C. One transformant was kept as SJ4587 (DN1885/pSJ4587).

### 4B. Construction of vector for integration of second expression cassette copy:

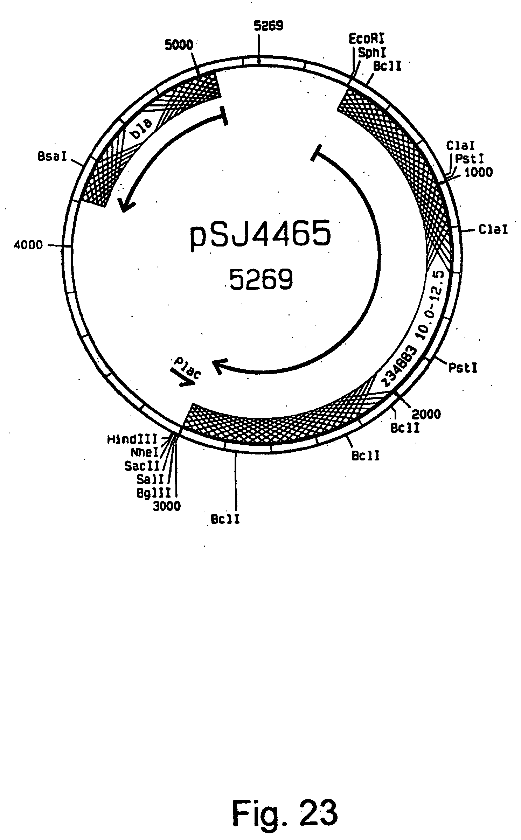

Plasmid pSJ4465 (figure 23) was constructed as follows:
Plasmid pSJ4459 was used as a substrate in a PCR reaction with primers #119882 and #119886, using 60 °C as annealing temperature and only 10 amplification cycles.
#119882: see above

#119886:
HindIII NheISacII SalI BglII← z34883 12565-12540 ---→
5'-GACTAAGCTTGCTAGCGGCGGAGCTCAGATCTGCCGCTGGATGTTAATGGCATCTGGC-3'

The amplified 2.6 kb fragment was digested with EcoRI and HindIII, and ligated to EcoRI + HindIII digested pUC19. The ligation mixture was transformed, by electroporation, into *E. coli* SJ2 selecting ampicillin resistance (200 µg/ml) on IPTG X-gal plates. Six white colonies were picked for plasmid preparation. Three were correct, and could be digested with enzymes EcoRI, HindIII, SphI, NheI, SacII, SalI and BglII. One transformant was kept as SJ4465 (SJ2/pSJ4465).

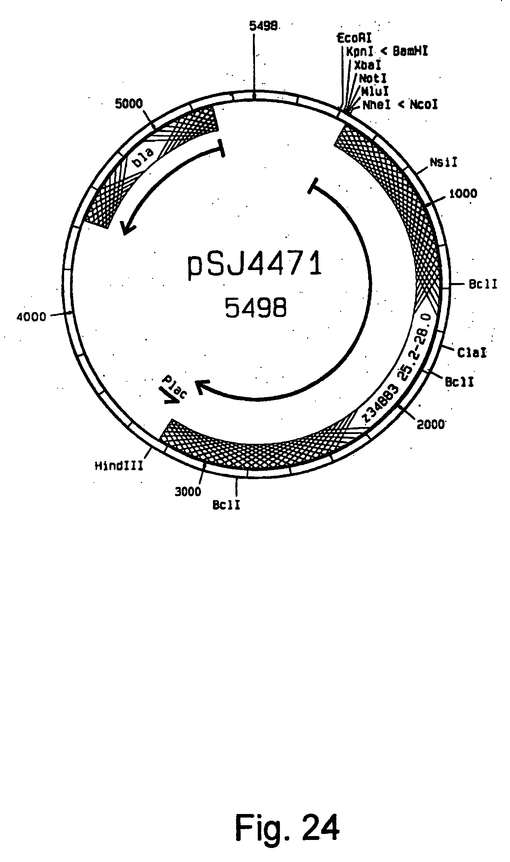

Plasmid pSJ4471 (figure 24) was constructed as follows: Plasmid pSJ4461 was used as a substrate in a PCR reaction with primers #119887 and #119888, using 60 °C as annealing temperature and only 10 amplification cycles.

#119887:
XbaI NotI MluI NheI NcoI ← z34883 25234-25260 ----
→
5'-
CAGTTCTAGAGCGGCCGCACGCGTGCTAGCCATGGCTTTTACAATAGAAGGAAAAGTCACCC-
3'

#119888:
HindIII<- z34883 28038-28015 →
5'-GACTAAGCTTCCTCTAACAGATTTCGAGGGCAG-3'

The amplified 2.8 kb fragment was digested with XbaI and HindIII, and ligated to XbaI + HindIII digested pUC19. The ligation mixture was transformed, by electroporation, into *E*. *coli* SJ2 selecting ampicillin resistance (200 µg/ml) on IPTG X-gal plates. 12 white colonies were picked for plasmid preparation. One was correct, and could be digested with enzymes XbaI, HindIII, NotI, MluI, NheI, and NcoI.
The strain was kept as SJ4471 (SJ2/pSJ4471).

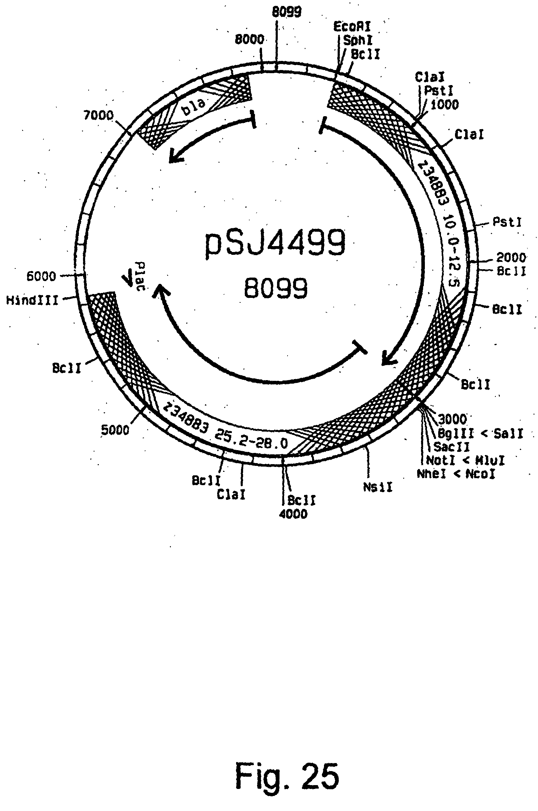

Plasmid pSJ4499 (figure 25) was constructed as follows:
Plasmid pSJ4465 was digested with EcoRI+NheI (+ with BsaI to further digest the unwanted part) and the 2.6 kb EcoRI-NheI fragment purified, from an agarose gel. The fragment was ligated to EcoRI + XbaI digested pSJ4471, and the ligation mixture transformed (electroporation) into *E. coli* SJ6 selecting ampicillin resistance (200 µg/ml). A correct transformant was kept as SJ4499 (SJ6/pSJ4499).

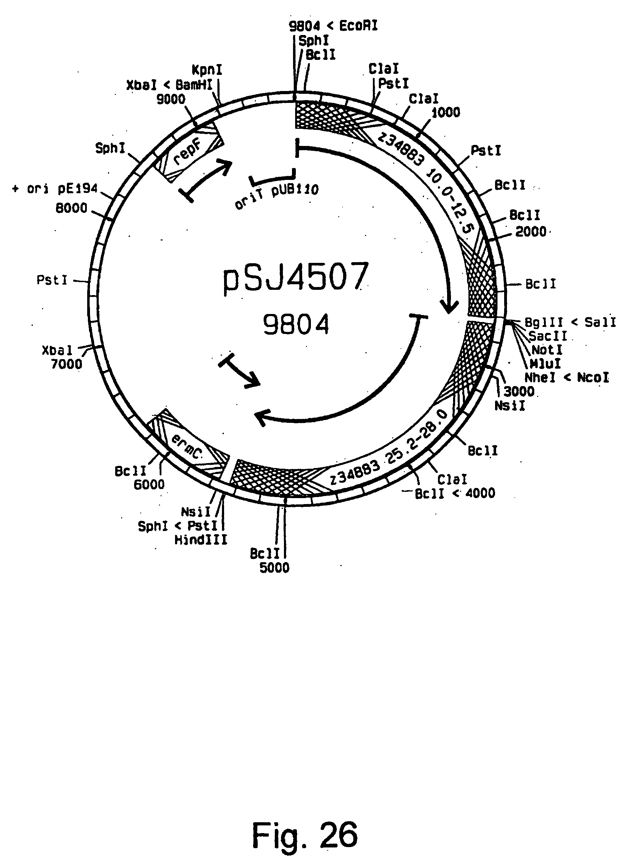

Plasmid pSJ4507 (figure 26) was constructed by digestion of pSJ4499 with EcoRI + HindIII, purification of the 5.4 kb fragment, and ligation to the 4.3 kb EcoRI-HindIII fragment of pSJ2739. The ligation mixture was transformed into *B*. *subtilis* DN1885 competent cells selecting erythromycin resistance (5 µg/ml) at 30 °C. One transformant was kept as SJ4507 (DN1885/pSJ4507).

### 4C. Insertion of a gene encoding Bacillus licheniformis alpha-amylase, amyL, into first and second integration vectors.

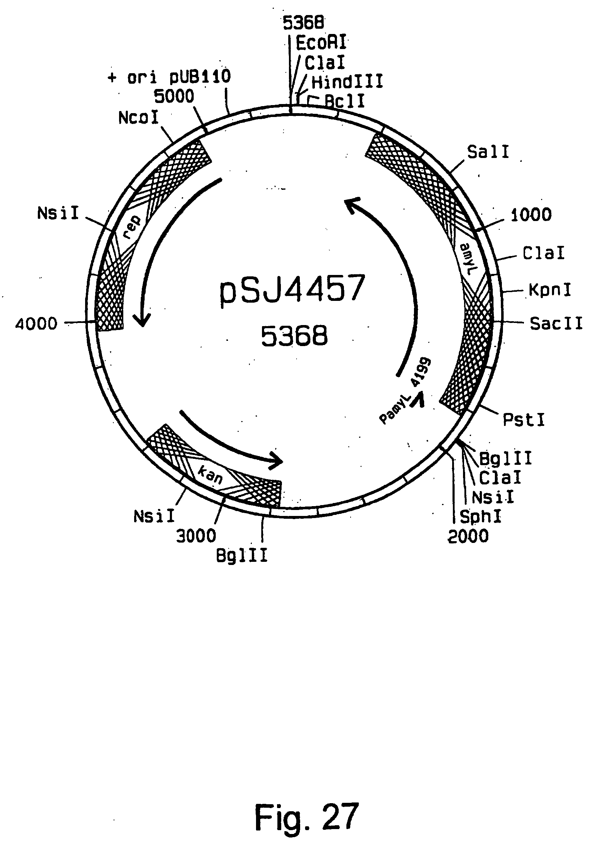

Plasmid pSJ4457 (figure 27) was constructed. This is almost identical to pSJ4277, previously described (figure 12) 'except for a few extra restriction sites upstream of the mutant *amyL* promoter. It was constructed by ligating the 0.2 kb SphI-PstI fragment purified from pSJ4338 (described in example 3A above, figure 13) to the 5 kb PstI-SphI fragment of pDN1981, and transformation of the ligation mixture into DN1885, selecting kanamycin (10 µg/ml) resistance. One transformant was kept as SJ4457 (DN1885/pSJ4457).

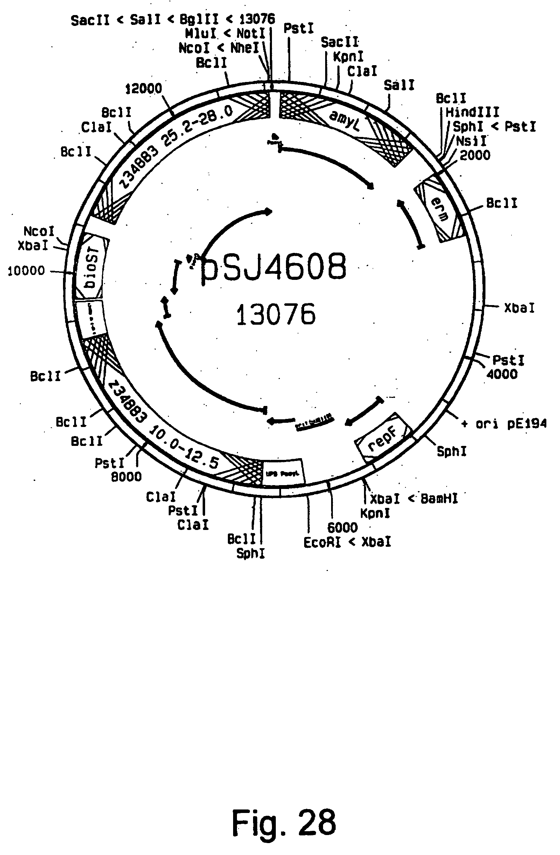

Plasmid pSJ4608 (figure 28) contains the *amyL* gene inserted into the first-copy integration vector, and was constructed as follows:
pSJ4457 was digested with BglII and HindIII, and the 1.9 kb fragment gel purified. This fragment was ligated to the 11.2 kb BglII-HindIII fragment from pSJ4587, and the ligation mixture transformed into competent DN1885 selecting erythromycin resistance (5 µg/ml) at 30 °C. An amylase-positive, GFP positive transformant was kept as SJ4608 (DN1885/pSJ4608).

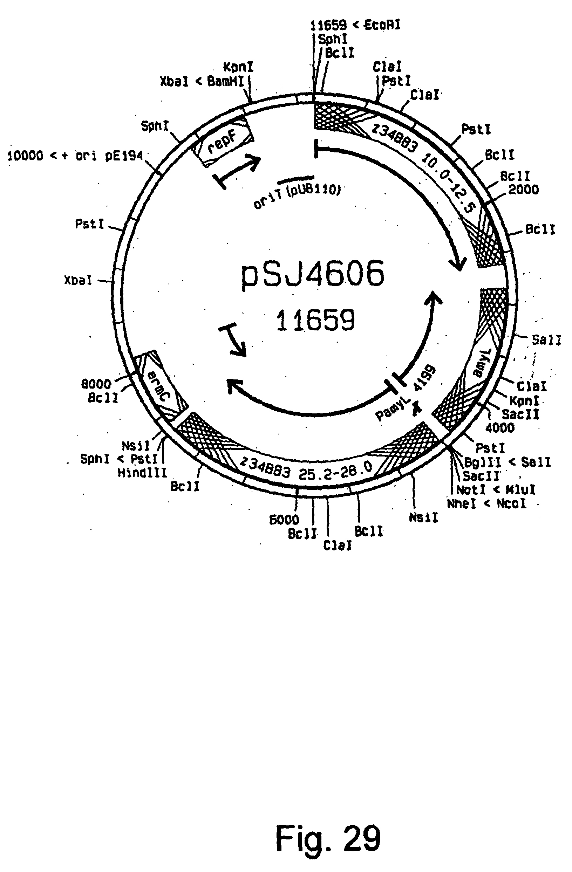

Plasmid pSJ4606 (figure 29) contains the *amyL* gene inserted into the second-copy integration vector, and was constructed as follows:
pSJ4457 was digested with BglII and BclI, and the 1.9 kb fragment gel purified. This fragment was ligated to BglII digested pSJ4507, and the ligation mixture transformed into competent DN1885 selecting erythromycin resistance (5 µg/ml) at 30 °C. A transformant containing the amyL gene inserted in the desired orientation was kept as SJ4606 (DN1885/pSJ4606).

### 4D. Transfer to Bacillus licheniformis and chromosomal integration of first integration vector.

Plasmid pSJ4608 was transformed into competent cells of the conjugative donor host strain PP289-5, selecting for erythromycin (5 µg/ml) and tetracycline (5 µg/ml) resistance on LBPSG plates supplemented with D-alanine (100 µg/ml), and a transformant kept as SJ4611 (PP289-5/pSJ4608).

Plasmid pSJ4608 was transferred into *B. licheniformis* SJ3047 (described en example 1) by conjugation as previously described. An amylase positive, GFP positive transconjugant was streaked on an LBPSG plate with erythromycin (5 µg/ml) and incubated at 50 °C overnight. Six amylase positive, GFP positive colonies were inoculated into individual 10 ml TY cultures and shaken at 30 °C. Cultures were then plated to single colonies on LBPSG at 30 °C, and plates replica plated to LBPSG with erythromycin (5 µg/ml). Putative erythromycin sensitive, amylase positive colonies were reisolated. The GFP positive phenotype was clearly visible after about one week on the plates. Two amylase positive, GFP positive and erythromycin sensitive strains were kept as SJ4629 and SJ4630.

### 4E. Transfer to Bacillus licheniformis and chromosomal integration of second integration vector.

Plasmid pSJ4606 was transformed into competent cells of the conjugative donor host strain PP289-5, selecting for erythromycin (5 µg/ml) and tetracycline (5 µg/ml) resistance on LBPSG plates supplemented with D-alanine (100 µg/ml), and a transformant kept as SJ4609.

Plasmid pSJ4606 was transferred into *B. licheniformis* strains SJ4629 and SJ4630 by conjugation from SJ4609. Two transconjugants from each recipient were streaked on erythromycin (5 µg/ml) plates and incubated at 50 °C overnight. 12 colonies appearing at 50 °C from each recipient were inoculated into individual 10 ml TY cultures and shaken at 30 °C. This propagation was repeated once. Cultures were then plated to single colonies on LBPSG at 30 °C, and plates replica plated to LBPSG with erythromycin (5 µg/ml).
Erythromycin sensitive, GFP negative colonies were obtained from 12 of the 24 cultures.
Some strains kept were SJ4669, SJ4670 and SJ4671 (from separate TY cultures, from SJ4629 recipient), and SJ4672, SJ4673 and SJ4674 (from separate TY cultures, from SJ4630 recipient).

### 4F. Test of two-copy strains.

Strain SJ4270 (the single-copy strain), SJ4629 and SJ4630 (single-copy strains expressing also GFP, and containing the *pps* gene segments inserted), and the two-copy strains SJ4669-SJ4674 were tested in BPX shake flasks, incubated at 37 °C for one week, and α-amylase activity measured. The yield is expressed in percentage of the yield obtained with the control one-copy strain, SJ4270.

| **Strain** | **Relative yield** |
|---|---|
| **SJ4270 (1)** | 100 |
| **SJ4270 (2)** | 94 |
| **SJ4629 (1)** | 78 |
| **SJ4629 (2)** | 75 |
| **SJ4630 (1)** | 47 |
| **SJ4630 (2)** | 47 |
| **SJ4669** | 163 |
| **SJ4670** | 159 |
| **SJ4671** | 159 |
| **SJ4672** | 141 |
| **SJ4673** | 143 |
| **SJ4674** | 143 |

The surprisingly low yield obtained with strain SJ4630 was presumably due to a later detected contamination of this strain.

Strain SJ4671, which by Southern analysis (see below) was confirmed to harbour the two copies of the *amyL* gene inserted as intended, in opposite orientation, was chosen for further analysis by fermentation in a laboratory fermentor. On day 4 of the fermentation, a sample of the SJ4671 culture was plated on LBPG plates with dyed amylopectin to check for the amylase phenotype. The 800 colonies appearing were all amylase positive to the same extent, reflecting the stability of the strain.

10 of these colonies were inoculated into BPX shake flasks, with SJ4671 from the frozen stock via a plate as control, with the following result (7 days, 37 °C).

| **Strain** | **Relative yield** |
|---|---|
| **SJ4671** | 100 |
| **Colony 1** | 81 |
| **Colony 2** | 94 |
| **Colony 3** | 94 |
| **Colony 4** | 88 |
| **Colony 5** | 102 |
| **Colony 6** | 96 |
| **Colony 7** | 92 |
| **Colony 8** | 106 |
| **Colony 9** | 88 |
| **Colony 10** | 90 |

The yield from SJ4671 in this experiment was 102 % of the yield of SJ4671 obtained in the first experiment .
Thus, the single colonies picked after fermentation are all giving yields equivalent to the yield from the original SJ4671 strain, reflecting **the stability** of the strain.

### 4G. Southern analysis.

Chromosomal DNA from strains SJ3047, SJ4270, SJ4629, and two-copy strains, including SJ4671, was extracted and analysed by southern blot hybridizations. DNA preparations were digested with HindIII, or with HindIII + KpnI, and the DNA fragments separated by agarose gel electrophoresis were transferred to Immobilon-N (Millipore) membrane by vacuum blotting. The membrane was probed with biotinylated plasmid pDN1981 DNA; using the NE-Blot Photope Kit and Photope Detection Kit from New England Biolabs.

Plasmid pDN1981 used as probe contains the entire Termamyl gene (on a 2.4 kb SphI-HindIII fragment), the pUB110 origin and *rep gene,* and the kanamycin resistance gene.

The resulting blots reveals from strain SJ3047 a 4.2 kb HindIII fragment, as expected.
From strain SJ4270, a 3.3 kb HindIII fragment is revealed.
From strain SJ4629, a 9.6 kb HindIII fragment is revealed.
From strain SJ4671, 10.6 kb HindIII fragment is revealed.
Finally, strain SJ4671 DNA digested with both HindIII and KpnI reveals fragments of 5.2 kb, 4.2 kb and 1.3 kb.
This hybridization pattern is as expected from the desired two-copy strain, confirming the correctness of strain SJ4671.

## Claims

1. A prokaryotic cell expressing a gene of interest **characterised in that**,
i) the cell comprises on the chromosome two anti-parallelly transcribed copies of said gene of interest; and
ii) any DNA segment situated between the two copies of the gene of interest under item i) only comprises a DNA sequence which is not essential for the growth of the cell.

2. A method for constructing a prokaryotic cell expressing a gene of interest comprising the construction of a cell wherein
i) the cell comprises on the chromosome two anti-parallelly transcribed copies of said gene of interest; and
ii) any DNA segment situated between the two copies of the gene of interest under item i) only comprises a DNA sequence which is not essential for the growth of the cell.

3. The prokaryotic cell according to claim 1, or the method according to claim 2, wherein said gene is a gene which is capable of expressing a polypeptide which are secreted from the cell.

4. The prokaryotic cell according to claims 1 or 3, or the method according to claims 2 or 3, wherein said gene encodes an enzyme.

5. The prokaryotic cell according to any of claims 1 or 3-4, or the method according to any of claims 2 or 3-4, wherein said prokaryotic cell is a Bacillus cell.

6. The prokaryotic cell according to any of claims 1 or 3-5, or the method according to any of claims 2 or 3-5, wherein said DNA segment, which is situated between two copies of said gene, is at least 10 bp long, more preferably from 10 bp to 6000 bp, and even more preferably from 75 bp to 4500 bp long.

7. The prokaryotic cell according to any of claims 1 or 3-6, or the method according to any of claims 2 or 3-6, wherein said DNA segment, which is situated between the two copies of the gene of interest, does not comprise an antibiotic resistance marker gene.

8. A method for production and isolation of a polypeptide of interest, comprising
i) culturing a prokaryotic cell according any of claims 1 or 3-7 under suitable conditions permitting expression of the polypeptide of interest encoded by the gene of interest; and
ii) isolating the polypeptide of interest.

9. The method for production and isolation of a polypeptide of interest of claim 8, wherein the culturing of a prokaryotic cell of item i) is done under conditions wherein the polypeptide of interest is expresses in an amount of at least 2 g polypeptide (dry matter) / kg culture medium.

10. The method for production and isolation of a polypeptide of interest of claim 8, wherein the culturing of a prokaryotic cell of item i) is in a fermentation process on a volume scale which is > 10 m³.

## Patentansprüche

1. Prokaryontische Zelle, die ein Gen von Interesse exprimiert, **dadurch gekennzeichnet, dass**
i) die Zelle am Chromosom zwei antiparallel transkribierte Kopien des Gens von Interesse umfasst, und
ii) ein zwischen zwei Kopien des Gens von Interesse unter Punkt i) liegendes DNA-Segment, das lediglich eine DNA-Sequenz umfasst, die für das Wachstum der Zelle nicht wesentlich ist.

2. Verfahren zum Aufbau einer prokaryontischen Zelle, das ein Gen von Interesse exprimiert, umfassend den Aufbau einer Zelle, wobei
i) die Zelle am Chromosom zwei antiparallel transkribierte Kopien des Gens von Interesse umfasst, und
ii) ein zwischen zwei Kopien des Gens von Interesse unter Punkt i) liegendes DNA-Segment, das lediglich eine DNA-Sequenz umfasst, die für das Wachstum der Zelle nicht wesentlich ist.

3. Prokaryontische Zelle nach Anspruch 1 oder Verfahren nach Anspruch 2, wobei das Gen ein solches Gen ist, das fähig ist, aus der Zelle abgegebene Polypeptide zu exprimieren.

4. Prokaryontische Zelle nach Anspruch 1 oder 3 oder Verfahren nach Anspruch 2 oder 3, wobei das Gen ein Enzym kodiert.

5. Prokaryontische Zelle nach einem der Ansprüche 1 oder 3-4 oder Verfahren nach einem der Ansprüche 2 oder 3-4, wobei die prokaryontische Zelle eine Bazilluszelle ist.

6. Prokaryontische Zelle nach einem der Ansprüche 1 oder 3-5 oder Verfahren nach einem der Ansprüche 2 oder 3-5, wobei das zwischen zwei Kopien des Gens liegende DNA-Segment mindestens 10 Bp lang, vorzugsweise 10 - 6000 Bp und am meisten bevorzugt 75 - 4500 Bp lang ist.

7. Prokaryontische Zelle nach einem der Ansprüche 1 oder 3-6 oder Verfahren nach einem der Ansprüche 2 oder 3-6, wobei das zwischen zwei Kopien des Gens von Interesse liegende DNA-Segment kein Markierungsgen einer antibiotischen Resistenz umfasst.

8. Verfahren zur Herstellung und Isolierung eines Polypeptids von Interesse, umfassend
i) Züchten einer prokaryontischen Zelle nach einem der Ansprüche 1 oder 3-7 unter geeigneten Bedingungen, welches Exprimieren des durch das Gen von Interesse kodierten Polypeptids von Interesse erlaubt, und
ii) Isolieren des Polypeptids von Interesse.

9. Verfahren zur Herstellung und Isolierung eines Polypeptids von Interesse des Anspruchs 8, wobei das Züchten einer prokaryontischen Zelle im Punkt i) unter solchen Bedingungen durchgeführt wird, dass das Polypeptid von Interesse in einer Menge von mindestens 2 g Polypeptid (Trockensubstanz)/kg Nährmedium exprimiert ist.

10. Verfahren zur Herstellung und Isolierung eines Polypeptids von Interesse des Anspruchs 8, wobei das Züchten einer prokaryontischen Zelle im Punkt i) ein Fermentierungsprozess einer Volumenskala von > 10 m³ ist.

## Revendications

1. Cellule procaryote exprimant un gène d'intérêt, **caractérisé en ce que**,
i) la cellule comprend sur le chromosome deux copies dudit gène d'intérêt, transcrites anti-parallèlement; et
ii) tout segment d'ADN situé entre les deux copies du gène d'intérêt selon le point i) ne comprend qu'une séquence d'ADN qui n'est pas essentielle à la croissance de la cellule.

2. Méthode pour construire une cellule procaryote exprimant un gène d'intérêt, comprenant la construction d'une cellule dans laquelle
i) la cellule comprend sur le chromosome deux copies dudit gène d'intérêt, transcrites anti-parallèlement; et
ii) tout segment d'ADN situé entre les deux copies du gène d'intérêt selon le point i) ne comprend qu'une séquence d'ADN qui n'est pas essentielle à la croissance de la cellule.

3. Cellule procaryote selon la revendication 1, ou la méthode selon la revendication 2, dans laquelle ledit gène est un gène qui est capable d'exprimer un polypeptide qui est sécrété par la cellule.

4. Cellule procaryote selon la revendication 1 ou 3, ou la méthode selon la revendication 2 ou 3, dans laquelle ledit gène code une enzyme.

5. Cellule procaryote selon l'une quelconque des revendications 1 ou 3 à 4, ou méthode selon l'une quelconque des revendications 2 ou 3 à 4, dans laquelle ladite cellule procaryote est une cellule de bacille.

6. Cellule procaryote selon l'une quelconque des revendications 1 ou 3 à 5, ou procédé selon l'une quelconque des revendications 2 ou 3 à 5, dans laquelle ledit segment d'ADN, qui est situé entre deux copies dudit gène a une longueur d'au moins 10 pb, de façon plus préférée de 10 pb à 6000 pb, et de façon encore plus préférée de 75 pb à 4500 pb.

7. Cellule procaryote selon l'une quelconque des revendications 1 ou 3 à 6, ou le procédé selon l'une quelconque des revendications 2 ou 3 à 6, dans laquelle ledit segment d'ADN, qui est situé entre les deux copies du gène d'intérêt, ne comprend pas de gène marqueur de résistance antibiotique.

8. Une méthode pour la production et l'isolement d'un polypeptide d'intérêt, qui comprend :
i) la mise en culture une cellule procaryote selon l'une quelconque des revendications 1 ou 3 à 7 dans des conditions appropriées permettant l'expression du polypeptide d'intérêt codé par le gène d'intérêt ; et
ii) l'isolement du polypeptide d'intérêt.

9. Méthode pour la production et l'isolement d'un polypeptide d'intérêt selon la revendication 8, dans laquelle la mise en culture d'une cellule procaryote selon le point i) s'effectue dans des conditions où le polypeptide d'intérêt est exprimé dans une quantité d'au moins 2 g de polypeptide (matière sèche)/kg de milieu de culture.

10. Méthode pour la production et l'isolement d'un polypeptide d'intérêt selon la revendication 8, dans laquelle la mise en culture d'une cellule procaryote selon le point i) se situe dans un processus de fermentation à une échelle de volume qui est > 10 m³.
